# EUROPÄISCHE PATENTANMELDUNG

(11) **EP 3 213 744 A1**
(43) Veröffentlichungstag der Anmeldung: **06.09.2017**
(21) Anmeldenummer: 16158111.1
(22) Anmeldetag: 01.03.2016
(51) Int. Cl.: A61K 9/00, A61K 47/10, A61K 47/14, A61K 31/192

(54) **GALENISCHE ZUBEREITUNG VON NSAID**

(71) Anmelder: CHEMISCHE FABRIK KREUSSLER & CO. GMBH, 65203 Wiesbaden (DE)
(72) Erfinder: Heydt-Lotter, Dr. Silke, 65201 Wiesbaden (DE); Lotter, Dr. Matthias, 65201 Wiesbaden (DE); Travers, Dr. Stephan, 65187 Wiesbaden (DE)
(74) Vertreter: dompatent von Kreisler Selting Werner - Partnerschaft von Patent- und Rechtsanwälten mbB

(57) **Zusammenfassung**

Die vorliegende Anmeldung betrifft eine galenische Zubereitung von nichtsteroidalen Antiphlogistika in Form eines Gels.

## Beschreibung

Die vorliegende Anmeldung betrifft eine galenische Zubereitung von nichtsteroidalen Antiphlogistika in Form eines Gels.

Nichtsteroidale Antirheumatika, welche auch als nichtsteroidale Antiphlogistika bezeichnet werden, sind Schmerzmittel. Sie hemmen Symptome von Entzündungsprozessen wie Schmerz und Schwellung. Die am häufigsten zu beobachtenden Nebenwirkungen sind Magenschleimhautschädigungen.

Aus diesem Grund begegnet die Verabreichung nichtsteroidaler Antiphlogistika (NSAID) zunehmend Bedenken des Verbrauchers. Neben der oralen Aufnahme besteht jedoch auch die Möglichkeit einer transdermalen Aufnahme. Die transdermale Aufnahme von NSAID in Form von Cremes, Gelen oder Sprays haben gegenüber der systemischen Schmerzbehandlung die Vorteile, dass die Konzentration im Blutplasma deutlich geringer ist, was zu einer besseren Verträglichkeit führt. Zudem wird der manuellen Behandlung (dem Einmassieren des Präparates) ein positiver psychologischer und schmerzreduzierender Effekt zugeschrieben. Weiterhin ist die Therapie kostengünstig.

Es hat sich jedoch gezeigt, dass insbesondere bei Gelen das Auftragen auf und Einmassieren in die Haut als unangenehm vom Verbraucher empfunden wird. Dies ist insbesondere darauf zurückzuführen, dass bei der manuellen Behandlung, also während des Einreibens, die Gele verklumpen, so dass sich Kügelchen auf der Haut ausbilden. Der durch ein Gel weiterhin hervorgerufene kühlende Effekt wird hierdurch vermindert. Insbesondere ist auch die Haptik hierdurch verschlechtert, was zu einer geringeren Compliance führen kann. Es besteht somit Bedarf an einer galenischen Zubereitung von NSAID, die auch beim Auftragen und Einreiben eine angenehme Haptik aufweist.

Überraschenderweise hat sich gezeigt, dass eine wässrige Zusammensetzung in Form eines Gels gemäß Anspruch 1 die Nachteile aus dem Stand der Technik vermeidet. Bevorzugte Ausführungsformen sind in den abhängigen Ansprüchen offenbart.

Das erfindungsgemäße Gel, also die erfindungsgemäße Zusammensetzung, weist Wasser in einem Anteil von 40 Gew.-% oder mehr auf. Wasser ist damit das wesentliche Lösungsmittel, so dass es sich bei dem Gel um eine wässrige Zusammensetzung handelt.

Ein Gel ist ein System, das aus mindestens zwei Komponenten besteht. Die feste Komponente, der Strukturbildner, bildet ein dreidimensionales Netzwerk, dessen Poren durch eine oder mehrere Flüssigkeiten ausgefüllt. Die flüssige Komponente ist dadurch in der festen immobilisiert. Beide Komponenten durchdringen sich vollständig. Bei Raumtemperatur behält das Gel ohne äußere Einwirkung seine vorgegebene Form. Es kann jedoch ohne größeren Kraftaufwand auf einer Oberfläche, beispielsweise auf der Haut eines Menschen, eingerieben werden.

Das erfindungsgemäße Gel weist wenigstens ein nichtsteroidales Antiphlogistikum auf. Dieses ist vorzugsweise ausgewählt aus Arylpropionsäure und deren Derivaten, Arylessigsäure und deren Derivaten, Indolessigsäure und deren Derivaten, sowie Oxicamen. Dabei kann das erfindungsgemäße Gel auch Mischungen der genannten Komponenten aufweisen. Insbesondere weist das Gel ein oder mehrere NSAID, ausgewählt aus Ibuprofen, Flurbiprofen, Naproxen, Ketoprofen und Tiaprofensäure auf. Diese können gut über die Haut aufgenommen werden, so dass ein für eine Wirkung ausreichender Wirkstoffgehalt erreicht werden kann. Besonders bevorzugt handelt es sich bei der wässrigen Zusammensetzung in Form eines Gels gemäß der vorliegenden Erfindung um ein solches für die transdermale Aufnahme von Ibuprofen, wobei Ibuprofen als einziges NSAID in der Zusammensetzung enthalten ist.

Die erfindungsgemäße Zusammensetzung umfasst das wenigstens eine NSAID in einer solchen Konzentration, dass eine schmerzlindernde Wirkung erreicht wird. Hohe Konzentrationen bewirken entsprechend höhere Wirkstoffspiegel im Zielgewebe. Dabei ist auch die von Patienten üblicherweise aufgetragene Menge zu berücksichtigen. Es hat sich gezeigt, dass ein Anteil von 1 Gew.-% bis 10 Gew.-% an NSAID üblicherweise ausreichend ist, um einen Wirkstoffspiegel im Zielgewebe, der eine schmerzlindernde Wirkung ermöglicht, zu erreichen.

Bevorzugt beträgt der Anteil 2 Gew.-% bis 8 Gew.-%, insbesondere 3 Gew.-% bis 7 Gew.-%, besonderes bevorzugt 5 Gew.-%, jeweils bezogen auf das Gesamtgewicht der Zusammensetzung von 100 Gew.-%. Gerade ein Anteil von 5 Gew.-% an NSAID ist der Kompromiss zwischen der Bereitstellung eines kostengünstigen Produktes einerseits und einem ausreichenden Wirkstoffspiegel andererseits, so dass dieser Wert und insbesondere der Bereich von 3 Gew.-% bis 7 Gew.-% für das NSAID bevorzugt ist.

Die erfindungsgemäße Zusammensetzung weist weiterhin wenigstens einen Strukturbildner in einem Anteil von 3 Gew.-% bis 40 Gew.-%, bezogen auf das Gesamtgewicht der Zusammensetzung von 100 Gew.-%, auf. Der Strukturbildner ist dabei für die Ausbildung des Gels verantwortlich und bildet hier das feste Netzwerk. Ein Anteil von 3 Gew.-% bis 40 Gew.-% ist dabei einerseits ausreichend und gleichzeitig notwendig, um die weiteren Komponenten in Lösung zu einem Gel zusammenzuhalten. Als Strukturbildner können an sich bekannte Polymere eingesetzt werden. Bevorzugt ist der Strukturbildner ein amphiphiles Blockcopolymer aus Ethylenoxid- und Propylenoxideinheiten. Es hat sich gezeigt, das entsprechende Blockcopolymere besonders stabile Netzwerke ausbilden, wobei jedoch gleichzeitig ein Gel erhalten wird, das gut auf die Haut aufzutragen und dort zu verteilen ist, ohne dass sich die Struktur des Gels ändert.

Bevorzugt wird ein Poloxamer als Strukturbildner eingesetzt. Poloxamere sind tensidartige Blockcopolymere mit einem zentralen Polypropylenglykolteil, der an beiden Kettenenden mit jeweils einem Makrogolanteil verknüpft ist. Besonders bevorzugt ist der Strukturbildner Poloxamer 407.

Die dreistellige Ziffernfolge definiert dabei den Massenanteil der Ethylenoxideinheiten. Die letzte Ziffer mit dem Faktor 10 multipliziert gibt etwa den relativen Massenanteil der Ethylenoxideinheiten in Prozent an. Die voranstehenden Ziffern codieren die relative Molekülmasse des Polypropylenglykol-Blockes.

Bevorzugt weist die Zusammensetzung den wenigstens einen Strukturbildner in einem Anteil von 5 Gew.-% bis 35 Gew.-%, insbesondere von 5 Gew.-% bis 30 Gew.-%, besonders von 7 Gew.-% bis 25 Gew.-% oder von 7 Gew.-% bis 20 Gew.-%, bevorzugt von 8 Gew.-% bis 15 Gew.-% oder von 8 Gew.-% bis 12 Gew.-%, jeweils bezogen auf das Gesamtgewicht der Zusammensetzung von 100 Gew.-%, auf.

Die erfindungsgemäße Zusammensetzung (das Gel) weist weiterhin wenigstens einen Lösungsvermittler in einem Anteil von 3 Gew.-% bis 40 Gew.-% auf. Es ist erfindungsgemäß möglich, dass die Zusammensetzung 2, 3 oder mehr Lösungsvermittler aufweist. Besonders bevorzugt weist sie 2 Lösungsvermittler auf. Dabei ist der wenigstens eine Lösungsvermittler ausgewählt aus der Gruppe bestehend aus Propylenglykol, Butylenglykolen, Glycerin, Pentaerythrit, Sorbit, Mannit, Oligosacchariden, Dimethylisosorbid, Isopropylmyristat, Monooleat von ethoxylierten Glyceriden mit 8 bis 10 Ethylenoxid-Einheiten, Polyethylenglykol 200 bis 600, Transcutol, Glycofurol und Cyclodextrinen, insbesondere aus Dimethylisosorbid und Isopropylmyristat.

Überraschendeerweise hat sich gezeigt, dass die Kombination aus Lösungsvermittler und Strukturbildner in den genannten Mengenbereichen dafür sorgt, dass eine stabile Gelstruktur erhalten wird, die beim Auftragen auf ein Einreiben in die Haut eine angenehme Haptik aufweist. Eine Bildung krümeliger Strukturen, wie im Stand der Technik sonst üblich, erfolgt hier gerade nicht.

Insbesondere weist die Zusammensetzung 2 Lösungsvermittler auf, wobei einer insbesondere dafür sorgt, dass das NSAID in dem weiterhin enthaltenen aliphatischen Alkohol besonders gut löslich ist. Eine weiterhin optional vorhandene Gruppe von Lösungsvermittlern ermöglicht eine bessere Verteilung auf der Haut. Bevorzugt weist die Zusammensetzung daher einen Lösungsvermittler auf, welcher die bessere Löslichkeit des NSAID ermöglicht, und einen Weiteren, der eine verbesserte Verteilung auf der Haut ermöglicht. Dimethylisosorbid ist dabei bevorzugt, um die verbesserte Löslichkeit des NSAID zu ermöglichen. Isopropylmyristat sorgt insbesondere für eine bessere Verteilung der Zusammensetzung auf der Haut und damit auch das Eindringen von NSAID gleichmäßig auf der Haut. Besonders bevorzugt umfasst die erfindungsgemäße Zusammensetzung daher 2 Lösungsvermittler, insbesondere Dimethylisosorbid und Isopropylmyristat.

In dieser bevorzugten Ausführungsform weist die Zusammensetzung zwei Lösungsvermittler auf. Bevorzugt ist das Gewichtsverhältnis des ersten Lösungsvermittlers, welcher die bessere Löslichkeit des NSAID ermöglicht, zum zweiten Lösungsvermittler, eine verbesserte Verteilung auf der Haut ermöglicht, 10 : 1 bis 2 : 1, insbesondere 7 : 1 bis 2 : 1 und bevorzugt 5 : 1 bis 3 : 1.

Bevorzugt umfasst die Zusammensetzung den wenigstens einen Lösungsvermittler, also 1, 2, 3 oder mehr Lösungsvermittler, in einem Anteil von 5 Gew.-% bis 35 Gew.-% oder von 5 Gew.-% bis 30 Gew.-%, besonders von 7 Gew.-% bis 25 Gew.-%, insbesondere von 7 Gew.-% bis 20 Gew.-%, bevorzugt von 8 Gew.-% bis 15 Gew.-%, jeweils bezogen auf das Gesamtgewicht der Zusammensetzung von 100 Gew.-%.

Es hat sich gezeigt, dass ein Anteil von 3 Gew.-% notwendig ist, um das wenigstens eine NSAID in Lösung zu bringen. Dabei ist der Anteil an Lösungsvermittlern abhängig von der Menge des eingesetzten NSAID. Dabei sind 7 Gew.-% bis 25 Gew.-% ausreichend, um NSAID in einem Anteil von 3 Gew.-% bis 7 Gew.-% in Lösung zu bringen. Besonders bevorzugt beträgt der Anteil an Lösungsvermittler 8 Gew.-% bis 15 Gew.-%.

Umfasst die erfindungsgemäße Zusammensetzung zwei verschiedene Lösungsvermittler, wie zuvor definiert, beträgt der Anteil des ersten Lösungsvermittlers vorzugsweise 2 Gew.-% bis 35 Gew.-%, insbesondere 5 Gew.-% bis 25 Gew.-%, bevorzugt 7 Gew.-% bis 14 Gew.-%. Der Anteil am zweiten Lösungsvermittler beträgt vorzugsweise 0,5 Gew.-% bis 10 Gew.-%, insbesondere von 1 Gew.-% bis 7 Gew.-%, besonders bevorzugt von 1,5 Gew.-% bis 5 Gew.-%, jeweils bezogen auf das Gesamtgewicht der Zusammensetzung von 100 Gew.-%.

Weiterhin weist die erfindungsgemäße Zusammensetzung wenigstens einen aliphatischen Alkohol in einem Anteil von 10 Gew.-% oder mehr auf. Der aliphatische Alkohol dient dabei als primäres Lösungsmittel für das NSAID, um dieses später zusammen mit Wasser in das Netzwerk des Strukturbildners einzubauen. Vorzugsweise weist der wenigstens eine aliphatische Alkohol die allgemeine Formel CₙH₂ₙ₊₁OH auf, wobei n eine ganze natürliche Zahl von 2 bis 10 ist. Bevorzugt ist der aliphatische Alkohol ausgewählt aus Ethanol, Butanol, Propanol und/oder Hexanol. Dabei sind hier alle Isomere der genannten Alkohole umfasst. Besonders bevorzugt ist der aliphatische Alkohol Isopropanol. In diesem sind NSAID und insbesondere das bevorzugte Ibuprofen besonders gut löslich.

Der Anteil des wenigstens einen Alkohols liegt vorzugsweise in einem Bereich von 10 Gew.-% bis 40 Gew.-%, insbesondere von 12 Gew.-% bis 30 Gew.-%, besonders bevorzugt von 15 Gew.-% bis 25 Gew.-%, jeweils bezogen auf das Gesamtgewicht der Zusammensetzung von 100 Gew.-%. Auch hier ist der Anteil an Alkohol abhängig vom Anteil des NSAID, welches in der Zusammensetzung enthalten ist. Der Anteil an Alkohol liegt immer unterhalb des Anteils an Wasser, so dass Wasser das Hauptlösungsmittel darstellt.

Um das NSAID im Gel gleichmäßig zu verteilen, beträgt das Gewichtsverhältnis von NSAID zum Strukturbildner vorzugsweise 1 : 10 bis 10 : 1, insbesondere von 1 : 5 bis 5 : 1 und besonders bevorzugt von 1 : 5 bis 1 : 3. Hierdurch wird eine besonders gleichmäßige Wirkstoffverteilung erreicht und auch die Struktur des Gels ist angenehm für den Verbraucher.

In einer besonders bevorzugten Ausführungsform betrifft die vorliegende Erfindung eine Zusammensetzung, die aus
a. Ibuprofen als nicht-steroidalem Antiphlogistikum in einem Anteil von 1 Gew. -% bis 10 Gew.-%, insbesondere 2 Gew.-% bis 8 Gew.-%, besonders 3 Gew.-% bis 7 Gew.-%, bevorzugt 5 Gew.-%,
b. Poloxamer 407 als Strukturbildner in einem Anteil von 3 Gew.-% bis 40 Gew.-%, insbesondere von 5 Gew.-% bis 35 Gew.-% oder von 5 Gew.-% bis 30 Gew.-%, besonders von 7 Gew.-% bis 25 Gew.-% oder von 7 Gew.-% bis 20 Gew.-%, bevorzugt von 8 Gew.-% bis 15 Gew.-% oder von 8 Gew.-% bis 12 Gew.-%,
c. Dimethylisosorbid (DMIS) und Isopropylmyristat (IPM) als Lösungsvermittler, wobei die Gesamtmenge an Lösungsvermittler von 3 Gew.-% bis 40 Gew.-%, insbesondere von 5 Gew.-% bis 35 Gew.-% oder von 5 Gew.-% bis 30 Gew.-%, besonders von 7 Gew.-% bis 25 Gew.-% oder von 7 Gew.-% bis 20 Gew.-%, bevorzugt von 8 Gew.-% bis 15 Gew.-% beträgt,
d. Isopropanol als alkoholisches Lösungsmittel in einem Anteil von wenigstens 10 Gew.-%, insbesondere von 10 Gew.-% bis 40 Gew.-%, besonders von 12 Gew.-% bis 30 Gew.-%, bevorzugt von 15 Gew.-% bis 25 Gew.-%, und
e. Wasser in einem Anteil von 40 Gew.-% oder mehr, besteht.

Soweit in der vorliegenden Anmeldung Gew.-% von Zusammensetzungen genannt sind, beziehen sie sich diese jeweils auf das Gesamtgewicht der Zusammensetzung, welches 100 Gew.-% beträgt.

In dieser bevorzugten Ausführungsform weist die Zusammensetzung Dimethylisosorbid und Isopropylmyristat als Lösungsvermittler auf. Bevorzugt ist das Gewichtsverhältnis von Dimethylisosorbid zu Isopropylmyristat 10 : 1 bis 2 : 1, insbesondere 7 : 1 bis 2 : 1 und bevorzugt 5 : 1 bis 3 : 1. Der Anteil an Dimethylisosorbid beträgt vorzugsweise 2 Gew.-% bis 35 Gew.-%, insbesondere 5 Gew.-% bis 25 Gew.-%, bevorzugt 7 Gew.-% bis 14 Gew.-%. Der Anteil an Isopropylmyristat beträgt vorzugsweise 0,5 Gew.-% bis 10 Gew.-%, insbesondere von 1 Gew.-% bis 7 Gew.-%, besonders bevorzugt von 1,5 Gew.-% bis 5 Gew.-%, jeweils bezogen auf das Gesamtgewicht der Zusammensetzung von 100 Gew.-%.

Die erfindungsgemäße Zusammensetzung kann nach an sich bekannten Herstellungsverfahren hergestellt werden. Die Formulierung hat den besonderen Vorteil, dass sich die Struktur beim Auftragen auf die Haut nicht verändert. Dabei bleibt die effektive Permeation des Wirkstoffes in die Haut verglichen mit im Stand der Technik bekannten Zusammensetzungen wenigstens erhalten. Weiterhin ist die erfindungsgemäße Formulierung lagerfähig, so dass auch eine länger andauernde Anwendung durch den Verbraucher ermöglicht wird.

Alle Angaben in % beziehen sich, soweit nicht anders angegeben, auf Gew.-%; Gew.-% beziehen sich dabei auf ein Gesamtgewicht der Zusammensetzung von 100 Gew.-%. Sind Verhältnisse angegeben, so ist hierunter das Gewichtsverhältnis der genannten Komponenten zu verstehen. Soweit der Begriff "umfassen" in der vorliegenden Anmeldung genannt ist, ist die Bedeutung von "bestehen aus" mit umfasst. Die zuvor genannten einzelnen Merkmale können erfindungsgemäß beliebig miteinander kombiniert werden.

### Ausführungsbeispiel:

Es wurde ein Gel mit der folgenden Zusammensetzung hergestellt.

| | |
|---|---|
| Ibuprofen | 5,0 Gew.-% |
| Isopropanol | 20,5 Gew.-% |
| Poloxamer 407 | 10,4 Gew.-% |
| Dimethylisosorbid | 7,5 Gew.-% |
| Isopropylmyristat | 2,0 Gew.-% |
| gereinigtes Wasser | 54,6 Gew.-% |

Das erhaltene Gel konnte angenehm auf die Haut aufgebracht werden, ohne dass dies zu Verklumpungen des Gels oder der Ausbildung eines unangenehm wirkenden Films auf der Haut führte. Das Gel war transparent, behielt bei Raumtemperatur die äußere Form und lies sich gut aus der Haut auftragen.

## Patentansprüche

1. Wässrige Zusammensetzung in Form eines Gels für die transdermale Aufnahme wenigstens eines nicht-steroidalen Antiphlogistikums (NSAID) umfassend:
a. wenigsten ein nicht-steroidales Antiphlogistikum in einem Anteil von 1 Gew.-% bis 10 Gew.-%,
b. wenigstens einen Strukturbildner in einem Anteil von 3 Gew.-% bis 40 Gew.-%,
c. wenigstens einen Lösungsvermittler in einem Anteil von 3 Gew.-% bis 40 Gew.-%,
d. wenigstens einen aliphatischen Alkohol in einem Anteil von 10 Gew.-% oder mehr und
e. Wasser in einem Anteil von 40 Gew.-% oder mehr,
wobei das Gesamtgewicht der Zusammensetzung 100 Gew.-% beträgt.

2. Zusammensetzung nach Anspruch 1, **dadurch gekennzeichnet, dass** sie zwei, drei oder mehr Lösungsvermittler, insbesondere zwei Lösungsvermittler aufweist.

3. Zusammensetzung nach Anspruch 1 oder 2, **dadurch gekennzeichnet, dass** sie ein oder mehrere nicht-steroidale Antiphlogistika, ausgewählt aus Arylpropionsäure und deren Derivaten, Arylessigsäure und deren Derivaten, Indolessigsäure und deren Derivaten, Anthranilsäure und deren Derivaten und Oxicamen, insbesondere ein oder mehrere, insbesondere ein nicht-steroidales Antiphlogistikum, ausgewählt aus Ibuprofen, Flurbiprofen, Naproxen, Ketoprofen und Tiaprofensäure, insbesondere Ibuprofen, aufweist.

4. Zusammensetzung nach einem der Ansprüche 1 bis 3, **dadurch gekennzeichnet, dass** sie das wenigstens eine NSAID in einem Anteil von 2 Gew.-% bis 8 Gew.-%, insbesondere von 3 Gew.-% bis 7 Gew.-%, bevorzugt 5 Gew.-%, bezogen auf das Gesamtgewicht der Zusammensetzung von 100 Gew.-%, aufweist.

5. Zusammensetzung nach einem der Ansprüche 1 bis 4, **dadurch gekennzeichnet, dass** der wenigstens eine Strukturbildner ein amphiphiles Blockcopolymer aus Ethylenoxid und Propylenoxid ist.

6. Zusammensetzung nach einem der Ansprüche 1 bis 5, **dadurch gekennzeichnet, dass** sie den wenigstens einen Strukturbildner in einem Anteil von 5 Gew.-% bis 35 Gew.-%, insbesondere von 5 Gew.-% bis 30 Gew.-%, besonders von 7 Gew.-% bis 25 Gew.-% oder von 7 Gew.-% bis 20 Gew.-%, bevorzugt von 8 Gew.-% bis 15 Gew.-% oder von 8 Gew.-% bis 12 Gew.-%, bezogen auf das Gesamtgewicht der Zusammensetzung von 100 Gew.-%, aufweist.

7. Zusammensetzung nach einem der Ansprüche 1 bis 6, **dadurch gekennzeichnet, dass** der wenigstens eine Lösungsvermittler ausgewählt ist aus der Gruppe bestehend aus Propylenglykol, Butylenglykolen, Glycerin, Pentaerythrit, Sorbit, Mannit, Oligosacchariden, Dimethylisosorbid, Isopropylmyristat, Monooleat von ethoxylierten Glyceriden mit 8 bis 10 Ethylenoxid-Einheiten, Polyethylenglykol 200 bis 600, Transcutol, Glycofurol und Cyclodextrinen, insbesondere aus Dimethylisosorbid und Isopropylmyristat.

8. Zusammensetzung nach einem der Ansprüche 1 bis 7, **dadurch gekennzeichnet, dass** sie den wenigstens einen Lösungsvermittler in einem Anteil von 5 Gew.-% bis 35 Gew.-% oder von 5 Gew.-% bis 30 Gew.-%, besonders von 7 Gew.-% bis 25 Gew.-%, insbesondere von 7 Gew.-% bis 20 Gew.-%, bevorzugt von 8 Gew.-% bis 15 Gew.-%, bezogen auf das Gesamtgewicht der Zusammensetzung von 100 Gew.-%, aufweist.

9. Zusammensetzung nach einem der Ansprüche 1 bis 8, **dadurch gekennzeichnet, dass** der wenigstens eine aliphatische Alkohol die allgemeine Formel CₙH₂ₙ₊₁OH aufweist, wobei n eine ganze natürliche Zahl von 2 bis 10 ist, insbesondere ist er ausgewählt aus Ethanol, Butanol, Propanol und/oder Hexanol, insbesondere Isopropanol.

10. Zusammensetzung nach einem der Ansprüche 1 bis 9, **dadurch gekennzeichnet, dass** sie den wenigstens einen Alkohol in einem Anteil von 10 Gew.-% bis 40 Gew.-%, insbesondere von 12 Gew.-% bis 30 Gew.-%, bevorzugt von 15 Gew.-% bis 25 Gew.-%, bezogen auf das Gesamtgewicht der Zusammensetzung von 100 Gew.-%, aufweist.

11. Zusammensetzung nach einem der Ansprüche 1 bis 10, **dadurch gekennzeichnet, dass** das Gewichtsverhältnis von NSAID zu Strukturbildner im Bereich von 1:10 bis 10:1, insbesondere von 1:5 bis 5:1 und besonders von 1:5 bis 1:3 liegt.

12. Zusammensetzung nach einem der Ansprüche 1 bis 11, **dadurch gekennzeichnet, dass** sie aus
a. Ibuprofen als nicht-steroidalem Antiphlogistikum in einem Anteil von 1 Gew. -% bis 10 Gew.-%, insbesondere 2 Gew.-% bis 8 Gew.-%, besonders 3 Gew.-% bis 7 Gew.-%, bevorzugt 5 Gew.-%,
b. Poloxamer 407 als Strukturbildner in einem Anteil von 3 Gew.-% bis 40 Gew.-%, insbesondere von 5 Gew.-% bis 35 Gew.-% oder von 5 Gew.-% bis 30 Gew.-%, besonders von 7 Gew.-% bis 25 Gew.-% oder von 7 Gew.-% bis 20 Gew.-%, bevorzugt von 8 Gew.-% bis 15 Gew.-% oder von 8 Gew.-% bis 12 Gew.-%,
c. Dimethylisosorbid (DMIS) und Isopropylmyristat (IPM) als Lösungsvermittler, wobei die Gesamtmenge an Lösungsvermittler von 3 Gew.-% bis 40 Gew.-%, insbesondere von 5 Gew.-% bis 35 Gew.-% oder von 5 Gew.-% bis 30 Gew.-%, besonders von 7 Gew.-% bis 25 Gew.-% oder von 7 Gew.-% bis 20 Gew.-%, bevorzugt von 8 Gew.-% bis 15 Gew.-% beträgt,
d. Isopropanol als alkoholisches Lösungsmittel in einem Anteil von wenigstens 10 Gew.-%, insbesondere von 10 Gew.-% bis 40 Gew.-%, besonders von 12 Gew.-% bis 30 Gew.-%, bevorzugt von 15 Gew.-% bis 25 Gew.-%, und
e. Wasser in einem Anteil von 40 Gew.-% oder mehr,
besteht, wobei das Gesamtgewicht der Zusammensetzung 100 Gew.-% beträgt.

13. Zusammensetzung nach Anspruch 12, **dadurch gekennzeichnet, dass** das Gewichtsverhältnis von Dimethylisosorbid (DMIS) zu Isopropylmyristat (IPM) 10:1 bis 2:1, insbesondere 7:1 bis 2:1 und bevorzugt 5:1 bis 3:1 beträgt.

14. Zusammensetzung nach Anspruch 12 oder 13, **dadurch gekennzeichnet, dass** der Anteil an Dimethylisosorbid (DMIS) von 2 Gew.-% bis 35 Gew.-%, insbesondere von 5 bis 25 Gew.-%, bevorzugt von 7 bis 14 Gew.-% beträgt.

15. Zusammensetzung nach einem der Ansprühe 12 bis 14, **dadurch gekennzeichnet, dass** der Anteil an Isopropylmyristat (IPM) 0,5 Gew.-% bis 10 Gew.-%, insbesondere von 1 Gew.-% bis 7 Gew.-%, bevorzugt von 1,5 Gew.-% bis 5 Gew.-% beträgt.
